# EUROPEAN PATENT SPECIFICATION

(11) **EP 4 429 741 B1**
(45) Date of publication and mention of the grant of the patent: **22.04.2026**
(21) Application number: 22813943.2
(22) Date of filing: 03.11.2022
(51) Int. Cl.: A61M 16/00, A61M 16/08, A61M 39/00

(54) **APPARATUS FOR ATTACHING INTUBATED NON-HUMAN ANIMAL TO GAS DELIVERY SYSTEM**
VORRICHTUNG ZUM ANSCHLIESSEN EINES INTUBIERTEN NICHT-MENSCHLICHEN TIERES AN EIN GASZUFUHRSYSTEM
APPAREIL PERMETTANT DE FIXER UN ANIMAL NON HUMAIN INTUBÉ À UN SYSTÈME DE DISTRIBUTION DE GAZ

(30) Priority: 10.11.2021 EP 21207638
(43) Date of publication of application: 18.09.2024
(73) Proprietor: Philip Morris Products S.A., 2000 Neuchâtel (CH)
(72) Inventor: TAN, Wei Teck, Redhill Singapore 159471 (SG); CHOOK, Win Yan, Redhill Singapore 159471 (SG); KHALID BIN ABDUL HALID, Danial, Redhill Singapore 159471 (SG); LAM, Sharon Ai Er, Redhill Singapore 159471 (SG); TOH, Wei Wen, Redhill Singapore 159471 (SG); YEO, Ying Shan, Redhill Singapore 159471 (SG); KOH, Jun Jia, Redhill Singapore 159471 (SG)
(74) Representative: Reddie & Grose LLP
(86) International application number: PCT/EP2022/080748
(87) International publication number: WO 2023/083701

(56) References cited:
- WO-A1-2020/150775
- US-A- 5 245 992
- US-A- 5 546 936
- US-A1- 2009 229 601
- US-B1- 8 707 950

## Description

The present disclosure relates to an apparatus for attaching an intubated non-human animal to a gas delivery system. The disclosure also relates to a gas delivery system incorporating such an apparatus.

Gas delivery systems are used to deliver gases or aerosols to subjects during preclinical in vivo testing. There are a limited number of commercially available gas delivery systems that are suitable for such testing. Intra-tracheal delivery involves the introduction of a substance directly into the trachea. Typically, intra-tracheal delivery involves the insertion of a tubular component, such as a needle or catheter, down the mouth and throat of a subject. Such a process is typically referred to as intra-tracheal intubation. The tubular component can maintain an open airway and serve as a conduit through which one or more gases or aerosols can be administered to the subject.

Due to the invasive nature of intra-tracheal intubation, discomfort can sometimes be imposed on the subject. As such, intubation is usually performed after administration of one or more of a general anaesthesia and a neuromuscular-blocking drug. Nevertheless, there remains a general desire to minimise one or more of stress and discomfort for an intubated subject, particularly during the process of connecting the subject to, and disconnecting the subject from, a gas delivery system.

US 2009/229601 A1 relates to an intubating airway device for supplying respiratory gas to a patient during an intubation process. The intubating airway device comprises a hollow tubular member, a rubber adaptor having an opening, a first connector, an airway tube and a second connector. The hollow tubular member comprises a proximal end, a distal end, and a port that extends outwardly from a peripheral surface of the hollow tubular member. The proximal end is coupled to the rubber adaptor and the distal end is detachably coupled to a first end portion of the first connector. The airway tube is detachably coupled to a second end portion of the first connector. The second connector is adapted to be detachably coupled to the first connector on detachment of the airway tube from the first connector.

US5245992A relates to tracheal tubes which are used to introduce gasses or vapors along the trachea, such as by an anesthesiologist during the administration of anesthesia to a patient undergoing surgery. In particular, this document relates to a tracheal tube which includes a flexible portion that allows for movement of the proximal end relative to the distal end of the tracheal tube without creating stress at the proximal or distal ends.

US5546936A relates to a specialized tracheal tube and a method of using such for introduction of gasses or vapors along the trachea, such as by an anesthesiologist during the administration of anesthesia to a patient undergoing surgery. In particular, this document relates to a tracheal tube having a filament reinforced flexible portion that allows for movement of the proximal end relative to the distal end of the tracheal tube without creating stress at the proximal or distal ends.

The invention is defined in the appended independent claim, to which reference should now be made. Optional features of the invention are defined in dependent claims. Aspects, embodiments or examples falling outside the scope of the appended independent claim are not part of the invention, and are merely included for illustrative or explanatory purposes.

The present disclosure provides an apparatus for attaching an intubated non-human animal to a gas delivery system. The apparatus may serve as a means for first allowing a non-human animal to be intubated, and then allowing the intubated non-human animal to be attached to a gas delivery system. The apparatus may also allow the intubated non-human animal to be detached from a gas delivery system. The apparatus may comprise a first component having a first end for attaching to an outlet of a gas delivery system. The apparatus may comprise a second component having a first end for intubating a non-human animal. The present disclosure also provides a gas delivery system incorporating such an apparatus.

The apparatus of the present disclosure may comprise a hollow tubular component connecting the first component to the second component to form a gas flow path within the apparatus. The gas flow path may extend from the first end of the first component to the first end of the second component. The hollow tubular component may be movable between at least: a first position and a second position. The first position may be a position, in which the first end of first component has a first orientation relative to the first end of second component. The second position may be a position, in which the first end of first component has a second orientation relative to the first end of second component.

The apparatus of the present disclosure may comprise a connecting mechanism positioned between the first component and the second component. The connecting mechanism may comprise: a first connecting member and a second connecting member. The first connecting member may be attached to a second end of the first component. The second connecting member may be attached to a second end of the second component. The first connecting member and the second connecting member may be configured to engage with one another to releasably connect the first component to the second component and form a gas flow path within the apparatus. The gas flow path may extend from the first end of the first component to the first end of the second component. The first connecting member and the second connecting member may be configured to connect to one another using no more than 180 degrees of rotation between the first connecting member and the second connecting member.

According to a first aspect of the disclosure, there is provided an apparatus for attaching an intubated non-human animal to a gas delivery system, the apparatus comprising: a first component having a first end for attaching to an outlet of a gas delivery system; a second component having a first end for intubating a non-human animal; and a hollow tubular component connecting the first component to the second component to form a gas flow path within the apparatus, the gas flow path extending from the first end of the first component to the first end of the second component, and wherein the hollow tubular component is movable between at least: a first position, in which the first end of first component has a first orientation relative to the first end of second component; a second position, in which the first end of first component has a second orientation relative to the first end of second component.

By providing an apparatus for attaching an intubated non-human animal to a gas delivery system where the apparatus comprises a hollow tubular component which is movable between the first and second positions specified above, the intubated non-human animal may be attached to the gas delivery system in an orientation which is not necessarily dependent on the orientation of the outlet of the gas delivery system to which the intubated non-human animal is attached. As such, the process of attaching the intubated non-human animal to the gas delivery system can be achieved in a manner which can help to minimise one or more of stress and discomfort for the intubated non-human animal. Given the limited number of suitable commercially available gas delivery systems, such an apparatus can offer a clear improvement over the art.

In more detail, in use, the apparatus of the first aspect of the disclosure may be used to intubate a non-human animal by inserting the first end of the second component into the mouth and throat of the non-human animal. This step may be conducted at a location suitable for such purposes, and this location may not necessarily be the location in which the gas delivery system resides. The apparatus may then be attached to the gas delivery system so that a gas can be delivered to the intubated non-human animal. This may involve placing the intubated non-human animal on a support surface and then attaching the first end of the first component to an outlet of the gas delivery system. Due to the movable configuration of the tubular element, the intubated non-human animal can be attached to the outlet of the gas delivery system without requiring precise alignment between the support surface and the outlet of the gas delivery system. The apparatus may therefore help the intubated non-human animal to be attached to the gas delivery system in a manner which can help to minimise one or more of stress and discomfort for the intubated non-human animal. Similar considerations may apply in the process of detaching the intubated non-human animal from the gas delivery system.

By providing the hollow tubular component as a distinct component from the first and second components, it is possible to achieve a moveable configuration for the apparatus without necessarily imposing a limitation on one or both of the material and design of each of the first component and the second component. That is, each of the first component and second component can be manufactured from a material and designed in a manner suited to their intended purpose. For example, the second component may be designed in a way which is particularly suitable for intubating the non-human animal, such as by forming the second component from a rigid tube. Likewise, the first component may be designed in a way which is particularly suitable for attaching to an outlet of a gas delivery system. This may include a need for achieving an air-tight seal with the outlet of the gas delivery system.

As discussed above, the hollow tubular component may provide a means for connecting the first component of the apparatus to the second component of the apparatus. The hollow tubular component may define a hollow internal space extending from a first end of the hollow tubular component to a second tubular component. This internal space may be referred to as the lumen of the hollow tubular component. A portion of the gas flow path of the apparatus may extend along the lumen of the hollow tubular component. That is, the hollow tubular component may be configured as a conduit for gas to flow from the first component of the apparatus to the second component of the apparatus.

The hollow tubular component is movable, for example relative to one or both of the first component and the second component, between at least a first position, in which the first end of first component has a first orientation relative to the first end of second component; and a second position, in which the first end of first component has a second orientation relative to the first end of second component. The hollow tubular component may therefore be a flexible tube. The hollow tubular component may be formed of a flexible material. Suitable flexible materials include one or more of: rubber, synthetic elastomers, phthalates, silicone, and combinations thereof.

The hollow tubular component may comprise a first portion connected to a second portion via one or more articulated joints. The one or more articulated joints may permit the first portion of the hollow tubular component to move relative to the second portion of the hollow tubular component. That is, movement of the first portion of the hollow tubular component relative to the second portion of the hollow tubular component about the one or more articulated joints may correspond to movement of the hollow tubular component between the first position and the second position.

The first portion of the hollow tubular component may be connected to the first component of the apparatus. The second portion of the hollow tubular component may be connected to the second component of the apparatus.

According to a second aspect of the disclosure, there is provided an apparatus for attaching an intubated non-human animal to a gas delivery system, the apparatus comprising: a first component having a first end for attaching to an outlet of a gas delivery system; a second component having a first end for intubating a non-human animal; and a connecting mechanism positioned between the first component and the second component, the connecting mechanism comprising: a first connecting member attached to a second end of the first component; and a second connecting member attached to a second end of the second component, wherein the first connecting member and the second connecting member are configured to engage with one another to releasably connect the first component to the second component and form a gas flow path within the apparatus, the gas flow path extending from the first end of the first component to the first end of the second component, and wherein the first connecting member and the second connecting member are configured to connect to one another using no more than 180 degrees of rotation between the first connecting member and the second connecting member.

Rotation of one connecting member relative to the other connecting member may be a desirable manner for connecting the first component of the apparatus to the second component of the apparatus, because it may provide a reliable means for achieving a secure connection between the two components. This may be desirable for improving the effectiveness of the gas flow path formed in the apparatus. Such a connection can be easy to implement. Such a connection may also be easy to reliably achieve on a repeat basis.

However, a rotational connection can also mean that at least some rotational stress is imposed on the intubated non-human animal when the intubated non-human animal is being attached to the gas delivery system. Therefore, by providing an apparatus for attaching an intubated non-human animal to a gas delivery system, where the apparatus comprises a connecting mechanism utilising no more than 180 of rotation, the process of attaching the intubated non-human animal to the gas delivery system can be achieved in a reliable manner, whilst also helping to limit one or more of stress and discomfort for the intubated non-human animal.

In particular, with the apparatus of the second aspect of the disclosure, the first component of the apparatus and the first connecting member of the connecting mechanism can be initially attached to an outlet of a gas delivery system. Separately, the second component of the apparatus and the second connecting member of the connecting mechanism can be used to intubate a non-human animal. This step may be conducted at a location suitable for such purposes, and this location may not necessarily be the location in which the gas delivery system resides.

The intubated non-human animal with the second component of the apparatus and the second connecting member of the connecting mechanism can then be brought to the outlet of a gas delivery system. The first and second connecting members can then engage with one another to releasably connect the first component to the second component, and thus releasably connect the intubated non-human animal to the gas delivery system. Since the first connecting member and the second connecting member are configured to connect to one another using no more than 180 degrees of rotation between the first connecting member and the second connecting member, the amount of rotational stress imposed on the intubated non-human animal can be restricted. This may help to minimise one or more of stress and discomfort for the intubated non-human animal during the process of attaching the non-human animal to the gas delivery system. This may also help to ease the manner in which the intubated non-human animal needs to be handled during the process of attaching the non-human animal to the gas delivery system; for example, in comparison to an arrangement having a threaded engagement requiring several multiples of 360 degrees of rotation.

The apparatus of the second aspect of the disclosure can therefore allow for a secure connection to be made between the intubated non-human animal and the gas delivery system, in a manner which can help to minimise one or more of stress and discomfort for the intubated non-human animal. Given the limited number of suitable commercially available gas delivery systems, such an apparatus can offer a clear improvement over the art.

The first connecting member and the second connecting member may be configured to connect to one another using no more than 135 degrees of rotation between the first connecting member and the second connecting member. The first connecting member and the second connecting member may be configured to connect to one another using no more than 90 degrees of rotation between the first connecting member and the second connecting member. The first connecting member and the second connecting member may be configured to connect to one another using no more than 45 degrees of rotation between the first connecting member and the second connecting member. Minimising the amount of rotation needed to form the connection between the first connecting member and the second connecting member may help to minimise the extent of any of stress and discomfort being imposed on the intubated non-human animal during the process of attaching the non-human animal to the gas delivery system.

The first connecting member and the second connecting member may be configured to connect to one another to form an airtight seal. The airtight seal may be formed around a portion of the gas flow path extending through the connecting mechanism.

The first connecting member and the second connecting member may be configured to connect to one another to form an airtight seal, using no more than 180 degrees of rotation between the first connecting member and the second connecting member. The first connecting member and the second connecting member may be configured to connect to one another to form an airtight seal, using no more than 135 degrees of rotation between the first connecting member and the second connecting member. The first connecting member and the second connecting member may be configured to connect to one another to form an airtight seal, using no more than 90 degrees of rotation between the first connecting member and the second connecting member. The first connecting member and the second connecting member may be configured to connect to one another to form an airtight seal, using no more than 45 degrees of rotation between the first connecting member and the second connecting member.

The first connecting member and the second connecting member may form a luer connection. The first connecting member and the second connecting member may each comprise one of a male luer connector and a female luer connector.

The first connecting member may comprise a male luer connector and the second connecting member may comprise a corresponding female luer connector.

The first connecting member may comprise a female luer connector and the second connecting member may comprise a corresponding male luer connector.

The first connecting member may comprise a female luer connector and the second connecting member may comprise a female luer connector. In such embodiments, the connecting mechanism may further comprise a double male luer connector arranged to connect the first connecting member to the second connecting member, the double male luer connector comprising a body having: a first male luer connector on one end of the body, the first male luer connector being configured to engage with the female luer connector of the first connecting member; and a second male luer connector on another end of the body, the second male luer connector being configured to engage with the female luer connector of the second connecting member. The body of the double male luer connector may be hollow. Such a double male luer connector arrangement may be advantageous, since one of the luer connector arrangements may be configured to create a connection using minimal rotation, such as a rotation of no more than 180 degrees.

The first connecting member may comprise a male luer connector and the second connecting member may comprise a male luer connector. In such embodiments, the connecting mechanism may further comprise a double female luer connector arranged to connect the first connecting member to the second connecting member, the double female luer connector comprising a body having: a first female luer connector on one end of the body, the first male luer connector being configured to engage with the male luer connector of the first connecting member; and a second female luer connector on another end of the body, the second female luer connector being configured to engage with the male luer connector of the second connecting member. The body of the double female luer connector may be hollow. Such a double female luer connector arrangement may be advantageous, since one of the luer connector arrangements may be configured to create a connection using minimal rotation, such as a rotation of no more than 180 degrees.

One such suitable double luer connector arrangement is that described in U.S. Patent Application Publication No. 2016/0206516 A1, the entire content of which is incorporated herein by reference thereto.

For example, a male luer connector described herein may comprise a tapered tubular portion configured to be received within a tubular receiving portion of a female luer connector. A female luer connector described herein may therefore comprise a tubular receiving portion configured to receive a portion of a male luer connector.

A double male luer connector described herein may include a first male luer connector on one end and a second male luer connector on the other end. The first male luer connector and the second male luer connector are in communication with each other. For example, gas may be able to flow through the first male luer connector and the second male luer connector.

An outer circumferential surface of the first male luer connector may constitutes a first tapered surface (conical surface) whose outer diameter decreases toward a leading end of the first male luer connector. An outer circumferential surface of the second male luer connector may constitute a second tapered surface (conical surface) whose outer diameter decreases toward a leading end of the second male luer connector. The double male luer connector may further include a tubular portion surrounding the first male luer connector. A female screw may be formed on an inner circumferential surface of the tubular portion.

The entirety of the double male luer connector may be integrally formed as one component.

The double male luer connector may further include a pair of engaging claws protruding outward at a location between the first male luer connector and the second male luer connector. The pair of engaging claws may be configured to engage with a corresponding engagement structure provided on a female luer connector, such as the female luer connector of the first connecting member or the female luer connector of the second connecting member. For example, the double male luer connector may be configured such that when the second male luer connector is inserted into a female luer connector, the pair of engaging claws engage with a corresponding engagement structure on the female luer connector. With this configuration, a secure connection between the second male luer connector and the female luer connector can be achieved with an airtight fit, whilst requiring relatively little rotation between the second male luer connector and the female luer connector.

The pair of engaging claws may protrude outward from the body of the double male luer connector. Such protrusion may extend in a radial direction away from the central axis of the double male luer connector. The body of the double male luer connector may comprise a flange that extends around the entire circumference of the body. The pair of engaging claws may be provided on a cylindrical, outer circumferential surface of the flange. Each engaging claw may have a surface which is inclined relative to the flange. Each engaging claw may have a surface which is inclined relative to the central axis of the double male luer connector. Such an inclined surface may help the engaging claws to form an airtight seal between the double male luer connector and a corresponding female luer connector when the double male luer connector is connected to the female luer connector.

The pair of engaging claws may be rotationally symmetrical with respect to the central axis of the double male luer connector. It will be appreciated that also the above paragraphs have primarily been described with reference to a double male luer connector, the disclosure therein may also be applicable to embodiments having a double female luer connector.

It will be appreciated that features described above in respect of the apparatus of the first aspect of the disclosure may be combined in any suitable manner with the apparatus of the second aspect of the disclosure. Likewise, features described above in respect of the apparatus of the second aspect of the disclosure may be combined in any suitable manner with the apparatus of the first aspect of the disclosure.

For example, the hollow tubular component may be positioned between the connecting mechanism and the first component. That is, the hollow tubular component may act to attach the first connecting member of the connecting mechanism to the second end of the first component. Alternatively, the hollow tubular component may be positioned between the connecting mechanism and the second component. That is, the hollow tubular component may act to attach the second connecting member of the connecting mechanism to the second end of the second component.

Further optional features of the apparatus of the present disclosure, including those of the first aspect and second aspect of the disclosure, will now be described.

The first end of the first component may comprise an opening defining a gas inlet for the apparatus.

The first component may comprise a hollow tube. The first component may consist of a hollow tube. The hollow tube may have a first end defining the first end of the first component and an opposed second end. The second end of the hollow tube may be attached to the hollow tubular component. The second end of the hollow tube may be attached to the first connecting member.

Where the first component comprises or consists of a hollow tube, the hollow tube may be rigid. That is, the first end of the hollow tube may not be moveable relative to the second end of the hollow tube. The hollow tube may be formed of a metal. The hollow tube may be formed of a plastic.

The first component may define a hollow internal space extending from a first end of the first component to a second end of the first component. This internal space may be referred to as the lumen of the first component. A portion of the gas flow path of the apparatus may extend along the lumen of the first component. That is, the first component may be configured as a conduit for gas to flow into the apparatus from the gas delivery system.

The first end of the second component may comprise an opening defining a gas outlet for the apparatus.

The second component may comprise a hollow tube. The second component may consist of a hollow tube. The hollow tube may have a first end defining the first end of the second component and an opposed second end. The second end of the hollow tube may be the second end of the second component.

The hollow tube of the second component may be rigid. That is, the first end of the hollow tube of the second component may not be moveable relative to the second end of the hollow tube of the second component.

Where the second component comprises or consists of a hollow tube, the hollow tube of the second component may be formed of metal. The hollow tube may be formed of a plastic.

The second component may comprise a catheter for intubating a non-human animal.

The first end of the second component may be bevelled.

The second component may define a hollow internal space extending from a first end of the second component to a second end of the second component. This internal space may be referred to as the lumen of the second component. A portion of the gas flow path of the apparatus may extend along the lumen of the second component. That is, the second component may be configured as a conduit for gas to flow out of the apparatus and into the intubated non-human animal.

As noted above, the present disclosure also includes a gas delivery system comprising an apparatus as described herein. Therefore, according to a third aspect of the present disclosure, there is provided a gas delivery system for delivering gas to at least one non-human animal, the system comprising: a body defining a gas chamber, the body having a gas chamber inlet configured to receive a supply of gas, and at least one gas chamber outlet; and an apparatus according to one or both of the first and second aspects of the disclosure, wherein said apparatus is configured to attach an intubated non-human animal to at least one gas chamber outlet of the body of the gas delivery system.

The gas delivery system may comprise a supply of a substance to be delivered to an intubated non-human animal attached to the apparatus. The supply may be attached to the gas chamber inlet such that the substance can be introduced to the gas chamber through the gas chamber inlet. The gas delivery system may therefore be configured to deliver the substance to an intubated non-human animal attached to the apparatus. The substance may be an aerosol, or may form an aerosol once entrained in the gas flow from the gas chamber inlet. The substance may comprise a gaseous drug.

The gas delivery system or systems described herein may be for delivering gas to at least one subject, for example a plurality of subjects. As such, the apparatus described herein may be an apparatus for attaching a subject to a gas delivery system. The or each subject may be a non-human animal such as a mouse. Thus, the gas delivery system may be for delivering gas to at least one non-human animal, for example a plurality of non-human animals. The system may be for delivering gas to a plurality of subjects, for example a plurality of non-human animals, simultaneously. As used herein, the term "subject" may be used to refer to a non-human animal.

As used herein, the term "engaged with" may be used to mean "directly engaged with". Engaged components may be connected, or attached, to each other. Engaged components may be in contact with each other. Engagement of two components may require no additional components such as adaptors or engagement means separate to the engaged components.

The technology will now be further described, by way of example only, with reference to the accompanying drawings in which:
Figure 1 shows a schematic view of an apparatus for attaching an intubated non-human animal to a gas delivery system;
Figure 2 shows a schematic view of a gas delivery system comprising a plurality of apparatus and
Figure 3 shows an enlarged view of a component of Figures 1 and 2.

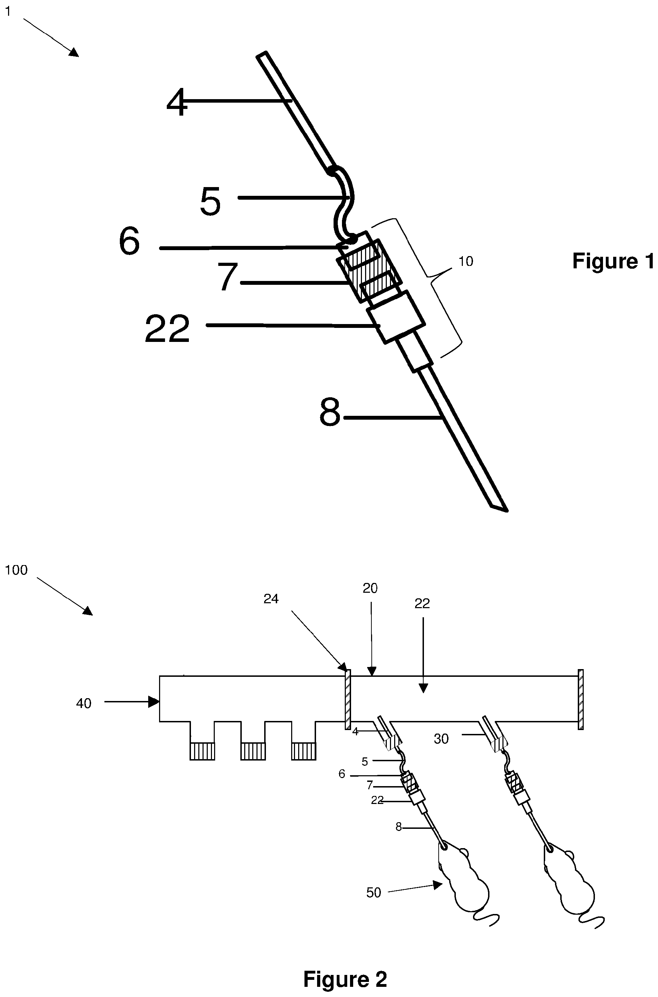

Figure 1 shows an apparatus 1 for attaching an intubated non-human animal to a gas delivery system. The apparatus 1 comprises a first component 4 in the form of a hollow metal tube. The first component 4 has a first end for attaching to an outlet of a gas delivery system. The apparatus also comprises a second component 8 in the form of a hollow metal tube or catheter. The second component 8 has a bevelled first end for intubating a non-human animal.

The apparatus further comprises a hollow tubular component 5 connecting the first component 4 to the second component 8. The hollow tubular component 5 is directly connected to the first component 4, for example, by way of an adhesive attaching a second end of the first component 4 to the hollow tubular component 5. The hollow tubular component 5 is indirectly attached to the second component 8 by way of a connecting mechanism 10, which will be described in more detail below. As such, a gas flow path exists in the apparatus, with the gas flow path extending from the first end of the first component 4 to the first end of the second component 8.

The hollow tubular component 5 is a flexible tube formed of one or more of: rubber, synthetic elastomers, phthalates, silicone, and combinations thereof. As such, the hollow tubular component 5 is movable between at least: a first position, in which the first end of first component 4 has a first orientation relative to the first end of second component 8; a second position, in which the first end of first component 4 has a second orientation relative to the first end of second component 8.

As indicated above, a connecting mechanism 10 resides between the first component 4 and the second component 8. The connecting mechanism 10 allows the first component 4 and the second component 8 to be releasably connected to one another to form the gas flow path in the apparatus 1.

The connecting mechanism 10 comprises a first connecting member 6 attached to the second end of the flexible tube 5. The first connecting member 6 is in the form of a female luer connector.

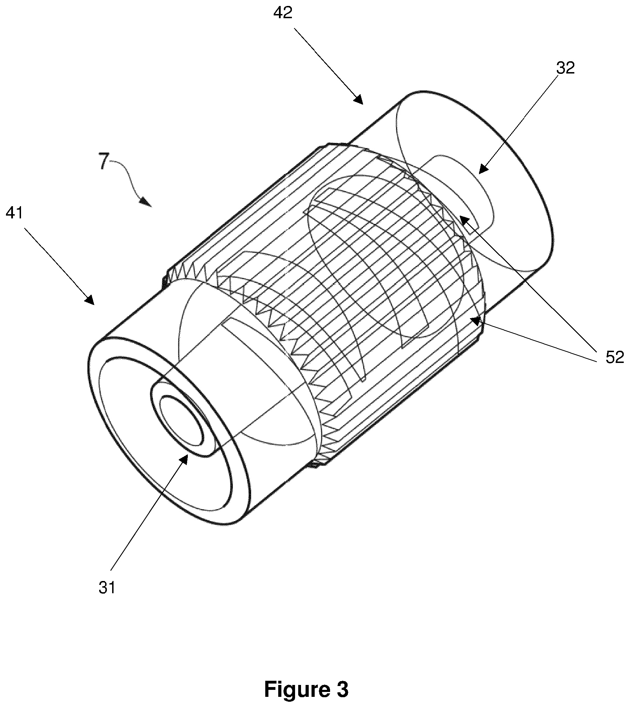

The connecting mechanism 10 also comprises a second connecting member in the form of a double male luer connector 7 and a second female luer connector 22. The second female luer connector 22 is attached to the second end of the second component 8, for example by way of an adhesive. The double male luer connector 7 is depicted in more detail in the perspective view of Figure 3. In Figure 3, the double male luer connector 7 is shown in partially transparent form for the purpose of enhancing the visibility of its features.

The first female luer connector 6 and the second female luer connector 22 are each configured to engage by way of rotation to form a secured connection with the double male luer connector 7. In particular, the double male luer connector 7 comprises a body having: a first male luer connector 31 on one end 41 of the body, and a second male luer connector 32 on another end 42 of the body. The first male luer connector 31 is configured to engage with the first female luer connector 6. The second male luer connector 32 is configured to engage with the second female luer connector 22. The second male luer connector 32 comprises a pair of outwardly protruding engaging claws 52. Each engaging claw has an inclined surface configured to engage with a corresponding engaging surface on the second female luer connector 22 to form an airtight fit with the second female luer connector 22. In particular, when the second male luer connector 32 and the second female luer connector 22 are brought together and rotated relative to one another, the inclined surfaces of the engaging claws 52 engage with corresponding surfaces of the second female luer connector 22 to form an airtight seal between the luer connectors. Said rotation between the luer connectors is no more than 180 degrees, such as 45 degrees.

Figure 2 depicts a gas delivery system 100 comprising two apparatus.

The gas delivery system 100 comprises a body 20 defining a gas chamber 22. The body 20 also has a gas chamber inlet 24 configured to receive a supply of gas from a supply 40 in fluid connection with the gas chamber inlet 24. The body 20 also has two gas chamber outlets 30 for allowing an intubated non-human animal to be attached to the body 20. In the embodiment of Figure 2, the intubated non-human animal is a mouse 50.

In use, a substance may be issued from the supply 40 into the gas chamber 22 through the gas chamber inlet 24. The substance may be an aerosol, or may form an aerosol once entrained in the gas flow from the gas chamber inlet 24. The substance may then be delivered to the intubated non-human animals 50 by passing out of the gas chamber outlets 30 and through the lumens formed in each apparatus 1 (i.e. through their respective gas flow paths).

## Claims

1. An apparatus (1) for attaching an intubated non-human animal (50) to a gas delivery system (100), the apparatus (1) comprising:
a first component (4) having a first end for attaching to an outlet (30) of a gas delivery system;
a second component (8) having a first end for intubating a non-human animal (50); and
a hollow tubular component (5) connecting the first component (4) to the second component (8) to form a gas flow path within the apparatus, the gas flow path extending from the first end of the first component to the first end of the second component, and
wherein the hollow tubular component (5) is movable between at least:
a first position, in which the first end of first component (4) has a first orientation relative to the first end of second component (8);
a second position, in which the first end of first component (4) has a second orientation relative to the first end of second component (8), and
wherein the apparatus (1) further comprises:
a connecting mechanism (10) positioned between the first component (4) and the second component (8), the connecting mechanism comprising:
a first connecting member (6) attached to a second end of the first component (4); and
a second connecting member (7) attached to a second end of the second component (8),
wherein the first connecting member (6) and the second connecting member (7) are configured to engage with one another to releasably connect the first component (4) to the second component (8) and form a gas flow path within the apparatus, the gas flow path extending from the first end of the first component (4) to the first end of the second component (8), and
wherein rotation of the first connecting member (6) relative to the second connecting member (7) connects the first component (4) to the second component (8), and
wherein the first connecting member (6) and the second connecting member (7) are configured to connect to one another using no more than 180 degrees of rotation between the first connecting member (6) and the second connecting member (7).

2. An apparatus (1) according to claim 1, wherein the hollow tubular component (5) comprises a flexible tube.

3. An apparatus (1) according to any preceding claim, wherein the hollow tubular component (5) comprises a first portion connected to a second portion via one or more articulated joints.

4. An apparatus (1) according to any preceding claim, wherein the first connecting member (6) and the second connecting member (7) are configured to connect to one another to form an airtight seal.

5. An apparatus (1) according to claim any preceding claim, wherein the first connecting member (6) and the second connecting member (7) each comprise one of a male luer connector and a female luer connector.

6. An apparatus (1) according to any preceding claim, wherein the first end of the first component (4) comprises an opening defining a gas inlet for the apparatus.

7. An apparatus (1) according to any preceding claim, wherein the first component (4) comprises a hollow tube, the hollow tube having a first end defining the first end of the first component and an opposed second end.

8. An apparatus (1) according to claim 7, wherein the second end of the hollow tube is attached to the hollow tubular component (5).

9. An apparatus (1) according to claim 7, wherein the second end of the hollow tube is attached to the first connecting member (6).

10. An apparatus (1) according to any preceding claim, wherein the first end of the second component (8) comprises an opening defining a gas outlet for the apparatus.

11. An apparatus (1) according to any preceding claim, wherein the second component (8) comprises a hollow tube, the hollow tube having a first end defining the first end of the second component and an opposed second end.

12. An apparatus (1) according to any preceding claim, wherein the second component (8) comprises a catheter for intubating a non-human animal (50).

13. An apparatus (1) according to any preceding claim, wherein the first end of the second component (8) is bevelled.

14. A gas delivery system (100) for delivering gas to at least one non-human animal (50), the system comprising:
a body (20) defining a gas chamber (22), the body having a gas chamber inlet (24) configured to receive a supply of gas, and at least one gas chamber outlet (30); and
an apparatus (1) according to any preceding claim, wherein the apparatus is configured to attach an intubated non-human animal (50) to the at least one gas chamber outlet (30) of the body (20) of the gas delivery system (100).

## Patentansprüche

1. Vorrichtung (1) zum Befestigen eines intubierten nichtmenschlichen Tieres (50) an einem Gasversorgungssystem (100), die Vorrichtung (1) aufweisend:
eine erste Komponente (4) mit einem ersten Ende zum Anbringen an einem Auslass (30) eines Gasversorgungssystems;
eine zweite Komponente (8) mit einem ersten Ende zum Intubieren eines nichtmenschlichen Tieres (50); und
eine hohle rohrförmige Komponente (5), welche die erste Komponente (4) mit der zweiten Komponente (8) verbindet, um einen Gasströmungsweg innerhalb der Vorrichtung zu bilden, wobei sich der Gasströmungsweg von dem ersten Ende der ersten Komponente zu dem ersten Ende der zweiten Komponente erstreckt, und
wobei die hohle röhrenförmige Komponente (5) zwischen wenigstens folgenden Positionen beweglich ist:
einer ersten Position, wobei das erste Ende der ersten Komponente (4) eine erste Ausrichtung in Bezug auf das erste Ende der zweiten Komponente (8) aufweist;
einer zweiten Position, wobei das erste Ende der ersten Komponente (4) eine zweite Ausrichtung in Bezug auf das erste Ende der zweiten Komponente (8) aufweist, und
wobei die Vorrichtung (1) ferner aufweist:
ein Verbindungsmechanismus (10), der zwischen der ersten Komponente (4) und der zweiten Komponente (8) positioniert ist, der Verbindungsmechanismus aufweisend:
ein erstes Verbindungselement (6), das an einem zweiten Ende der ersten Komponente (4) angebracht ist; und
ein zweites Verbindungselement (7), das an einem zweiten Ende der zweiten Komponente (8) angebracht ist,
wobei das erste Verbindungselement (6) und das zweite Verbindungselement (7) dazu ausgelegt sind, miteinander in Eingriff zu kommen, um die erste Komponente (4) lösbar mit der zweiten Komponente (8) zu verbinden und einen Gasströmungsweg innerhalb der Vorrichtung zu bilden, wobei sich der Gasströmungsweg von dem ersten Ende der ersten Komponente (4) zu dem ersten Ende der zweiten Komponente (8) erstreckt, und
wobei eine Drehung des ersten Verbindungselements (6) in Bezug auf das zweite Verbindungselement (7) die erste Komponente (4) mit der zweiten Komponente (8) verbindet, und
wobei das erste Verbindungselement (6) und das zweite Verbindungselement (7) dazu ausgelegt sind, unter Verwendung von nicht mehr als 180 Grad Drehung zwischen dem ersten Verbindungselement (6) und dem zweiten Verbindungselement (7) miteinander verbunden zu werden.

2. Vorrichtung (1) nach Anspruch 1, wobei die hohle rohrförmige Komponente (5) ein flexibles Rohr umfasst.

3. Vorrichtung (1) nach einem beliebigen vorhergehenden Anspruch, wobei die hohle rohrförmige Komponente (5) einen ersten Abschnitt aufweist, der über ein oder mehrere Gelenkverbindungen mit einem zweiten Abschnitt verbunden ist.

4. Vorrichtung (1) nach einem beliebigen vorhergehenden Anspruch, wobei das erste Verbindungselement (6) und das zweite Verbindungselement (7) dazu ausgelegt sind, miteinander verbunden zu werden, um eine luftdichte Abdichtung zu bilden.

5. Vorrichtung (1) nach einem beliebigen vorhergehenden Anspruch, wobei das erste Verbindungselement (6) und das zweite Verbindungselement (7) jeweils entweder einen Luer-Stecker oder eine Luer-Muffe aufweisen.

6. Vorrichtung (1) nach einem beliebigen vorhergehenden Anspruch, wobei das erste Ende der ersten Komponente (4) eine Öffnung aufweist, die einen Gaseinlass für die Vorrichtung definiert.

7. Vorrichtung (1) nach einem beliebigen vorhergehenden Anspruch, wobei die erste Komponente (4) ein Hohlrohr aufweist, wobei das Hohlrohr ein erstes Ende, welches das erste Ende der ersten Komponente definiert, und ein gegenüberliegendes zweites Ende aufweist.

8. Vorrichtung (1) nach Anspruch 7, wobei das zweite Ende des Hohlrohrs an der hohlen rohrförmigen Komponente (5) befestigt ist.

9. Vorrichtung (1) nach Anspruch 7, wobei das zweite Ende des Hohlrohrs an dem ersten Verbindungselement (6) befestigt ist.

10. Vorrichtung (1) nach einem beliebigen vorhergehenden Anspruch, wobei das erste Ende der zweiten Komponente (8) eine Öffnung aufweist, die einen Gasauslass für die Vorrichtung definiert.

11. Vorrichtung (1) nach einem beliebigen vorhergehenden Anspruch, wobei die zweite Komponente (8) ein Hohlrohr aufweist, wobei das Hohlrohr ein erstes Ende, welches das erste Ende der zweiten Komponente definiert, und ein gegenüberliegendes zweites Ende aufweist.

12. Vorrichtung (1) nach einem beliebigen vorhergehenden Anspruch, wobei die zweite Komponente (8) einen Katheter zum Intubieren eines nichtmenschlichen Tieres (50) aufweist.

13. Vorrichtung (1) nach einem beliebigen vorhergehenden Anspruch, wobei das erste Ende der zweiten Komponente (8) abgeschrägt ist.

14. Gasversorgungssystem (100) zur Versorgung wenigstens eines nichtmenschlichen Tieres (50) mit Gas, das System aufweisend:
ein Gehäuse (20), das eine Gaskammer (22) definiert, wobei das Gehäuse einen Gaskammereinlass (24) zum Aufnehmen einer Gasversorgung und wenigstens einen Gaskammerauslass (30) aufweist; und
eine Vorrichtung (1) nach einem beliebigen vorhergehenden Anspruch, wobei die Vorrichtung dazu ausgelegt ist, ein intubiertes nichtmenschliches Tier (50) an den wenigstens einen Gaskammerauslass (30) des Gehäuses (20) des Gasversorgungssystems (100) anzukoppeln.

## Revendications

1. Appareil (1) pour attacher un animal non humain intubé (50) à un système de distribution de gaz (100), l'appareil (1) comprenant :
un premier composant (4) ayant une première extrémité pour l'attachement à une sortie (30) d'un système de distribution de gaz ;
un deuxième composant (8) ayant une première extrémité pour intuber un animal non humain (50) ; et
un composant tubulaire creux (5) reliant le premier composant (4) au deuxième composant (8) pour former un trajet d'écoulement de gaz à l'intérieur de l'appareil, le trajet d'écoulement de gaz s'étendant de la première extrémité du premier composant à la première extrémité du deuxième composant, et
dans lequel le composant tubulaire creux (5) est mobile entre au moins :
une première position, dans laquelle la première extrémité du premier composant (4) a une première orientation par rapport à la première extrémité du deuxième composant (8) ;
une deuxième position, dans laquelle la première extrémité du premier composant (4) a une deuxième orientation par rapport à la première extrémité du deuxième composant (8), et
et dans lequel l'appareil (1) comprend en outre :
un mécanisme de raccordement (10) positionné entre le premier composant (4) et le deuxième composant (8), le mécanisme de raccordement comprenant :
un premier organe de raccordement (6) attaché à une deuxième extrémité du premier composant (4) ; et
un deuxième organe de raccordement (7) attaché à une deuxième extrémité du deuxième composant (8),
dans lequel le premier organe de raccordement (6) et le deuxième organe de raccordement (7) sont configurés pour s'engager l'un avec l'autre pour connecter de manière libérable le premier composant (4) au deuxième composant (8) et former un trajet d'écoulement de gaz à l'intérieur de l'appareil, le trajet d'écoulement de gaz s'étendant de la première extrémité du premier composant (4) à la première extrémité du deuxième composant (8), et
dans lequel la rotation du premier organe de raccordement (6) par rapport au deuxième organe de raccordement (7) connecte le premier composant (4) au deuxième composant (8), et
dans lequel le premier organe de raccordement (6) et le deuxième organe de raccordement (7) sont configurés pour se connecter l'un à l'autre en n'utilisant pas plus de 180 degrés de rotation entre le premier organe de raccordement (6) et le deuxième organe de raccordement (7).

2. Appareil (1) selon la revendication 1, dans lequel le composant tubulaire creux (5) comprend un tube flexible.

3. Appareil (1) selon l'une quelconque des revendications précédentes, dans lequel le composant tubulaire creux (5) comprend une première partie raccordée à une deuxième partie par l'intermédiaire d'un ou plusieurs joints articulés.

4. Appareil (1) selon l'une quelconque des revendications précédentes, dans lequel le premier organe de raccordement (6) et le deuxième organe de raccordement (7) sont configurés pour se connecter l'un à l'autre pour former un joint étanche à l'air.

5. Appareil (1) selon l'une quelconque des revendications précédentes, dans lequel le premier organe de raccordement (6) et le deuxième organe de raccordement (7) comprennent chacun un connecteur Luer mâle ou un connecteur Luer femelle.

6. Appareil (1) selon l'une quelconque des revendications précédentes, dans lequel la première extrémité du premier composant (4) comprend une ouverture définissant une entrée de gaz pour l'appareil.

7. Appareil (1) selon l'une quelconque des revendications précédentes, dans lequel le premier composant (4) comprend un tube creux, le tube creux ayant une première extrémité définissant la première extrémité du premier composant et une deuxième extrémité opposée.

8. Appareil (1) selon la revendication 7, dans lequel la deuxième extrémité du tube creux est attachée au composant tubulaire creux (5).

9. Appareil (1) selon la revendication 7, dans lequel la deuxième extrémité du tube creux est attachée au premier organe de raccordement (6).

10. Appareil (1) selon l'une quelconque des revendications précédentes, dans lequel la première extrémité du deuxième composant (8) comprend une ouverture définissant une sortie de gaz pour l'appareil.

11. Appareil (1) selon l'une quelconque des revendications précédentes, dans lequel le deuxième composant (8) comprend un tube creux, le tube creux ayant une première extrémité définissant la première extrémité du deuxième composant et une deuxième extrémité opposée.

12. Appareil (1) selon l'une quelconque des revendications précédentes, dans lequel le deuxième composant (8) comprend un cathéter pour intuber un animal non humain (50).

13. Appareil (1) selon l'une quelconque des revendications précédentes, dans lequel la première extrémité du deuxième composant (8) est biseautée.

14. Système de distribution de gaz (100) pour distribuer du gaz à au moins un animal non humain (50), le système comprenant :
un corps (20) définissant une chambre de gaz (22), le corps ayant une entrée de la chambre de gaz (24) configurée pour recevoir une alimentation en gaz, et au moins une sortie de la chambre de gaz (30) ; et
Appareil (1) selon l'une quelconque des revendications précédentes, dans lequel l'appareil est configuré pour attacher un animal non humain intubé (50) à au moins une sortie de la chambre de gaz (30) du corps (20) du système de distribution de gaz (100).
